# EUROPEAN PATENT APPLICATION

(11) **EP 4 806 496 A1**
(43) Date of publication of application: **16.09.2026**
(21) Application number: 25195811.2
(22) Date of filing: 13.08.2025
(51) Int. Cl.: A61M 60/109, A61M 60/232, A61M 60/38, A61M 60/419, A61M 60/845

(54) **PUMP DRIVING DEVICE**

(30) Priority: 14.03.2025 CN 202510306178
(71) Applicant: Lifemotion Medical Technology Co., Ltd., Shenzhen, Guangdong 518102 (CN)
(72) Inventor: YE, Jianping, Shenzhen Guangdong, 518102 (CN); LIU, Yang, Shenzhen Guangdong, 518102 (CN); LI, Mingtao, Shenzhen Guangdong, 518102 (CN)
(74) Representative: Pfenning, Meinig & Partner mbB

(57) **Abstract**

The present invention provides a pump driving device, a shielding member is sleeved on an outer portion of a first opening of a housing to form a sleeving portion, a first space is reserved on the housing inside the shielding member, a first gap is provided in the sleeving portion between the housing and the shielding member, so that the first space is in communication with the first gap, a rotating member rotates in the first space to disturb the airflow, accelerating the flow of internal gas, and the first cavity is in communication with the first space. In this way, gas sequentially enters the first cavity and the first space from the second opening, and is discharged through the first gap, thereby forming an effective airflow heat dissipation channel. Moreover, the first gap is located outside the main heat source, the rotating member, thereby improving heat dissipation efficiency.

## Description

### CROSS REFERENCE TO RELATED APPLICATION

The present application claims priority to Chinese Patent Application No. 202510306178.7, filed March 14, 2025, the entire disclosure of which is hereby incorporated by reference.

### TECHNICAL FIELD

The embodiments of the present invention relates to the technical field of medical equipment, and in particular to a pump driving device.

### BACKGROUND ART

In clinical emergency treatment of critically ill patients with severe cardiopulmonary failure, ECMO (Extracorporeal Membrane Oxygenation) equipment is used to provide continuous extracorporeal respiratory and circulatory support for the patient, in order to gain more valuable time for emergency treatment. The core components of the ECMO equipment are an artificial lung (also known as membrane lung or oxygenator) and an artificial heart (also known as blood pump or power pump). With the continuous improvement of artificial lung and artificial heart technologies, ECMO equipment can operate for a longer period of time, which provides the prerequisite for the application of ECMO equipment in the treatment of patients with cardiopulmonary failure. The blood flow is mainly achieved by driving the centrifugal pump head to rotate. The pump driving device is crucial in the entire ECMO equipment, and therefore, the reliability of the pump driving device is one of the important factors for the stable operation of the ECMO equipment.

When the existing pump driving device operates at full load power for a long time, it tends to experience excessive temperature and insufficient heat dissipation. If the temperature of the pump driving device is too high, the heat will be transferred to the connected pump head, which may cause certain damage to the blood inside the pump head.

ECMO equipment usually has outdoor transportation requirements. Adverse weather conditions outdoors often cause the equipment to be rained on, and when liquid seeps into the equipment, it can cause equipment failure. If such failure occurs during use, it can seriously endanger the patient's life. The commonly used waterproof measure is the sealed type, which seals the entire device to prevent liquid infiltration, but this leads to insufficient heat dissipation. This is especially problematic in the field of ECMO, where the requirements are more stringent. Alternatively, heat dissipation can be achieved by optimizing the air channels, but waterproof performance is then difficult to meet the requirements of outdoor transportation.

### SUMMARY OF THE INVENTION

To this end, the embodiments of the present invention provide a pump driving device to solve the technical problem that a pump driving device used in ECMO equipment cannot simultaneously meet the requirements of heat dissipation and waterproofing.

In order to achieve the above object, the embodiments of the present invention provide the following technical solution:

According to a first aspect of the embodiments of the present invention, the present application provides a pump driving device, the device comprising: a housing, which is hollow inside to form a first cavity, and has a first opening and a second opening respectively formed at two ends thereof; a shielding member, which is sleeved on an outer side of the first opening and forms a sleeving portion with the housing, with a slot provided at an outer side of a top of the shielding member for fixing a pump head, and a first space is reserved inside the shielding member on top of the housing; and a rotating member, configured to disturb an airflow by rotation within the first space, with the first cavity and the first space being in communication, where a first gap is formed between the housing and the shielding member at the sleeving portion, and a plurality of recessed channels are provided on an outer side of the sleeving portion of the housing; gas enters the first cavity and the first space sequentially from the second opening, and is then discharged through the first gap and the plurality of recessed channels.

Furthermore, the rotating member comprises: a stator, mounted in a mounting slot within the first cavity, with a side of the stator in contact with an inner surface of the first cavity; a rotor, rotatably mounted within a second cavity formed by the stator being hollow, with a third gap formed between the rotor and an inner wall of the second cavity; a motor shaft, inserted into a third cavity formed by the rotor being hollow, with both ends of the motor shaft extending from the third cavity sleeved with bearings; and a rotating platform, mounted at a top end of the motor shaft and located within the first space below the slot.

Furthermore, a second gap is formed between bottoms of the plurality of recessed channels and the shielding member; and the first space is in communication with the first gap and the second gap.

Furthermore, the plurality of recessed channels extend along a junction between the stator and an inner surface of the first cavity and are arranged around the first cavity, with each recessed channel being in communication with the first gap and the first space.

Furthermore, an elastic member is provided between the stator and the mounting slot, and the elastic member is configured to undergo a deformation upon being compressed.

Furthermore, a motor blocking member is provided at an end where the stator and the rotor are away from the mounting slot, the motor blocking member covers the first opening, and the motor blocking member is configured to confine the stator within the first cavity.

Furthermore, the rotating platform comprises: an adapting portion, located on a side of the rotating platform near the slot, the adapting portion being internally provided with at least one magnetic member; a blade assembly, located on a side of the rotating platform away from the slot, having a plurality of arc-shaped blades; a first receiving portion, located in a middle portion of the rotating platform, and having a first through hole for receiving the top end of the motor shaft; and a first extending portion, formed by extending outward from a junction between the adapting portion and the blade assembly, a fourth gap being formed between an outer end of the first extending portion and an inner wall of the shielding member.

Furthermore, the motor blocking member comprises: a first body, located below the rotating platform and having a fifth gap between the first body and bottom ends of the blades;

a docking portion, provided at an edge of the first body and extending toward the housing, with a first step being formed at a docking position with an outer end of the first opening; a second receiving portion, located in a middle portion of the first body, having a second through hole for receiving the first receiving portion, and a protruding portion formed extending toward the first cavity around a periphery of the second through hole, where the protruding portion encloses a second space for accommodating the bearings, and a plurality of through slots are provided on an outer periphery of the protruding portion; and a second extending portion, formed by extending outward from an edge of the first body at a junction with the docking portion, where a sixth gap is formed between an outer end of the second extending portion and the inner wall of the shielding member, and the docking portion and the second extending portion form a second step and a first annular groove.

Furthermore, a sealing member is provided between the housing and the shielding member, and the sealing member covers the first gap.

Furthermore, a height of the first gap is smaller than a first distance between a bottom of the sealing member and the bottoms of the recessed channels, and the first distance is smaller than a height of the second extending portion.

Furthermore, a second distance between the bottoms of the recessed channels and a central axis of the first space is greater than or equal to a third distance between a bottom of the first annular groove and the central axis of the first space.

Furthermore, an arc-shaped transition section is provided on an outer side of the housing at an end of the bottom of each recessed channel facing the second opening, and the sealing member is provided between the arc-shaped transition section and the first opening.

Furthermore, the pump driving device further comprises: a closing member and a cover plate, the closing member is mounted to cover the second opening at a bottom of the pump driving device, the cover plate is arranged outside the closing member, a third space with an outward opening is formed between the closing member and the cover plate, at least one protrusion facing the third space is arranged on the closing member and an inner surface of the cover plate to form an annular flow guide channel, and a sum of a height of one protrusion on one side and a height of another protrusion on another side is greater than a fourth distance between the closing member and the cover plate.

Furthermore, a side surface of the protrusion is provided with an outward protruding portion.

Furthermore, the closing member comprises: a bottom panel, configured to cover the second opening, with a vent hole provided on the bottom panel; and a side panel, formed by folding an edge of the bottom panel outward, with an outer edge of the side panel connected to an outer end of the second opening.

Furthermore, the protrusion comprises: a first annular protrusion, the first annular protrusion surrounds an outer edge of the vent hole, an outer end of the first annular protrusion is folded and extends toward the side panel to form a second annular protrusion, and the first annular protrusion having the second annular protrusion and the bottom panel form a second annular groove.

Furthermore, the side panel comprises an inner side plate and an outer side plate, and the protrusion further comprises a third annular protrusion formed by an outer end of the inner side plate being folded inward and extending, the third annular protrusion is disposed opposite to the second annular protrusion; the inner side plate having the third annular protrusion and the bottom panel form a third annular groove; and the second annular groove and the third annular groove are connected via the bottom panel to form a first annular recess.

Furthermore, the protrusion further comprises a fourth annular protrusion formed by an inner side surface of the cover plate extending inward, the fourth annular protrusion surrounds an outer side of the first annular protrusion, the fourth annular protrusion extends into the first annular recess to form an in-recess extending portion, the in-recess extending portion is folded and extends toward the third annular groove to form a fifth annular protrusion, the fourth annular protrusion having the fifth annular protrusion and the cover plate form a fourth annular groove, and the fourth annular groove and the outer side plate having the third annular protrusion are disposed oppositely to form a second annular recess.

Furthermore, the in-recess extending portion is folded and extends toward the second annular groove to form a sixth annular protrusion, and the fourth annular protrusion having the sixth annular protrusion and the cover plate form a third annular recess.

Furthermore, the inner side plate, the outer side plate, and the fourth annular protrusion are all obliquely arranged on a side close to the first annular protrusion.

Furthermore, a seventh gap is formed between an outer end of the fifth annular protrusion and the inner side plate, and an eighth gap is formed between an outer end of the second annular protrusion and the fourth annular protrusion.

Furthermore, a transition plate is provided between the inner side plate and the outer side plate, one end of the transition plate is butted with an inner end of the outer side plate, and another end of the transition plate is connected to an outer side of the inner side plate, and the outer side plate, the transition plate, and the inner side plate together enclose a fifth annular groove, and a splash pad is provided in the fifth annular groove.

Furthermore, a width of the fourth annular groove is greater than a height of the fifth annular protrusion, the height of the fifth annular protrusion is greater than a fifth distance between an outer end of the fifth annular protrusion and an outer end of the third annular protrusion, and the fifth distance is greater than a height of the third annular protrusion.

Compared with the existing technology, the pump driving device provided in the embodiments of the present application forms a sleeving portion by sleeving a shielding member on the outer side of the first opening of the housing, and a first space is reserved and formed on the housing inside the shielding member. A first gap is provided in the sleeving portion between the housing and the shielding member, so that the first space is in communication with the first gap, and the sleeving portion is provided with a plurality of recessed channels; a rotating component rotates within the first space to disturb the airflow and accelerate the internal gas flow, and the first cavity is in communication with the first space. In this way, gas sequentially enters the first cavity and the first space from the second opening and is discharged through the first gap, thereby forming an effective airflow heat dissipation channel. Furthermore, the position of the first gap is located on the outer side of the main heat source rotating component, which improves heat dissipation efficiency. In addition, the first gap, the recessed channels, and the first space can also form a water drainage channel, thereby meeting both the requirements of heat dissipation and waterproofing.

A sleeving portion is formed by sleeving a shielding member on the outer side of the first opening of the housing, and a first space is reserved and formed on the housing inside the shielding member. A first gap and a second gap are provided in the sleeving portion between the housing and the shielding member, so that the first space is in communication with the first gap and the second gap. A rotating component rotates within the first space to disturb the airflow and accelerate the internal gas flow, and the first cavity is in communication with the first space. In this way, gas sequentially enters the first cavity and the first space from the second opening and is discharged through the first gap and each recessed channel, thereby forming an effective airflow heat dissipation channel. Furthermore, the positions of the first gap and the second gap are located on the outer side of the main heat source rotating component, which improves heat dissipation efficiency. In addition, the first gap, the recessed channels, and the first space also form a water drainage channel, thereby meeting both the requirements of heat dissipation and waterproofing.

### Brief Description of the Drawings

In order to more clearly illustrate the technical solutions in the embodiments of the present invention or in the existing technology, the accompanying drawings required in the description of the embodiments or the existing technology will be briefly introduced below. Obviously, the drawings described below are merely exemplary, and a person skilled in the art can also derive other drawings of embodiments based on the provided drawings without any creative effort.

The structures, proportions, sizes, etc., shown in the drawings of this specification are only used to cooperate with the contents disclosed in the specification to facilitate understanding and reading by a person skilled in the art, and are not intended to limit the implementation conditions of the present invention. Therefore, they have no technical substantive significance. Any structural modifications, changes in proportional relationships, or adjustments in size, as long as they do not affect the efficacy and objectives that the present invention can achieve, shall all fall within the scope covered by the technical content disclosed in the present invention.
FIG. 1 is a schematic diagram of the overall structure of a pump driving device provided in an embodiment of the present invention.
FIG. 2 is a schematic exploded view of the structure of a pump driving device provided in an embodiment of the present invention.
FIG. 3 is a side view schematic diagram of the structure of a pump driving device provided in an embodiment of the present invention.
FIG. 4 is a sectional view along the A-A direction of the pump driving device provided in an embodiment of the present invention as shown in FIG. 3.
FIG. 5 is a schematic enlarged view showing the structure of part I of the pump driving device provided in an embodiment of the present invention as shown in FIG. 4.
FIG. 6 is a schematic enlarged view showing the structure of part II of the pump driving device provided in an embodiment of the present invention as shown in FIG. 4.
FIG. 7 is a sectional view of the housing of a pump driving device provided in an embodiment of the present invention.
FIG. 8 is a sectional view of a stator of a pump driving device provided in an embodiment of the present invention.
FIG. 9 is a sectional view of a rotor of a pump driving device provided in an embodiment of the present invention.
FIG. 10 is a sectional view of a rotating platform of a pump driving device provided in an embodiment of the present invention.
FIG. 11 is a sectional view of a motor blocking member of a pump driving device provided in an embodiment of the present invention.

### Description of the Embodiments

The following specific embodiments are provided to illustrate the embodiments of the present invention. A person skilled in the art can easily understand other advantages and effects of the present invention based on the disclosure of this specification. Obviously, the described embodiments are part of the embodiments of the present invention, and not all of the embodiments. All other embodiments obtained by a person skilled in the art based on the embodiments of the present invention without creative work shall fall within the scope of protection of the present invention.

ECMO devices are usually required for outdoor transport. In order to prevent liquid from seeping into the device when it gets wet in harsh weather, a fully enclosed design is commonly used to seal the entire device. However, this is not conducive to heat dissipation of the device. ECMO devices have very stringent requirements for heat dissipation. According to the industry standard GB9706.1-2020 "Medical electrical equipment, Part 1: General requirements for basic safety and essential performance," as shown in Table 24, when the standard is converted, the temperature at the centrifugal pump head must not exceed 43°C. However, extreme environmental temperatures can reach 40°C. That is, under the condition of an ambient temperature of 40°C and full-load operation of the device, the temperature at the centrifugal pump head must not exceed 43°C, leaving a total temperature difference of only 3°C. It can be seen that under such extreme heat dissipation requirements, it is relatively difficult to further meet waterproofing requirements for ECMO devices.

An object of the embodiments of the present application is: on the one hand, to solve the technical problem that the pump driving device used for the ECMO device needs to have better heat dissipation, and on the other hand, to solve the technical problem of partial waterproof requirements of the device.

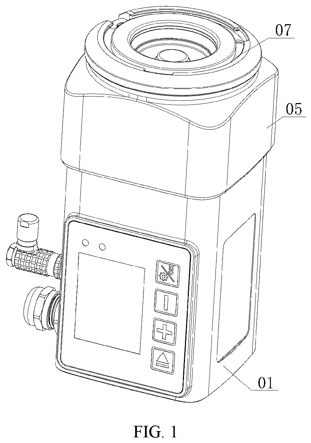

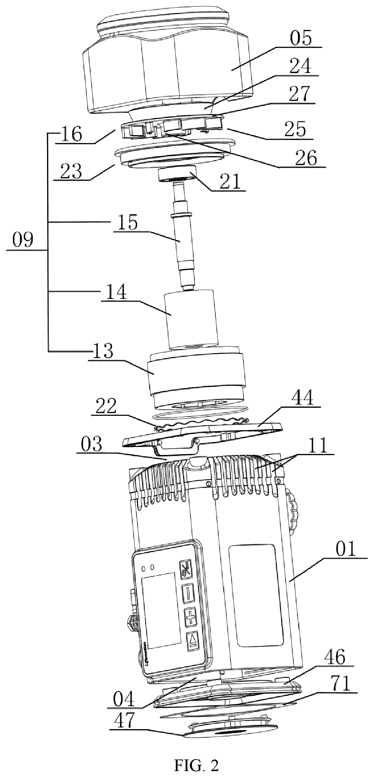

To solve the above technical problems, as shown in FIG. 1 and FIG. 2, the embodiments of the present application provide a pump driving device, specifically including: a housing 01, a shielding member 05, and a rotating member 09.

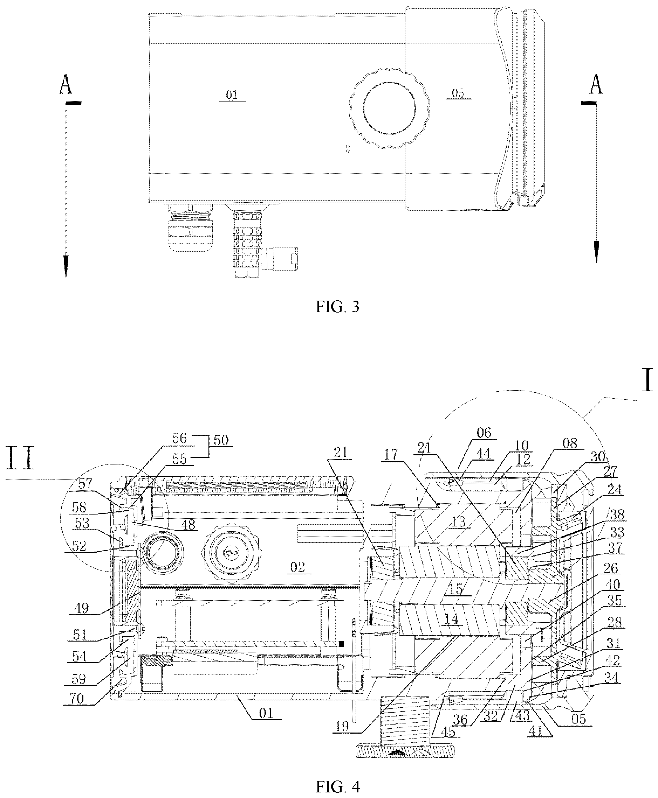

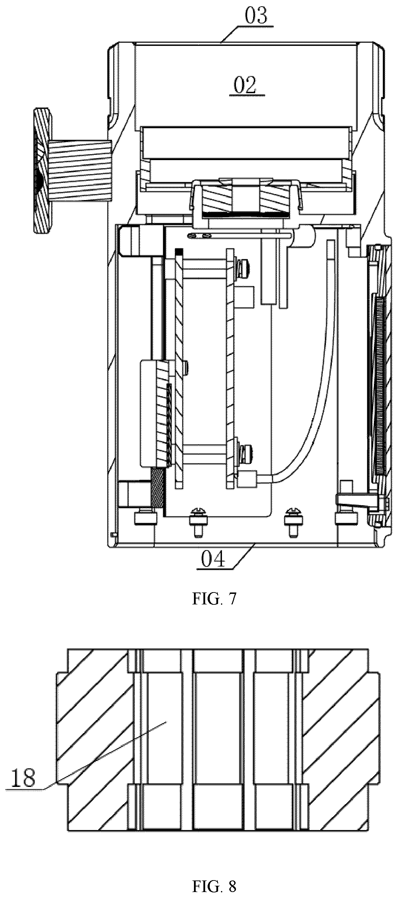

With reference to FIG. 3, FIG. 4, and FIG. 7, a first cavity 02 is formed as a hollow interior of the housing 01, and a first opening 03 and a second opening 04 are respectively formed at two ends of the housing 01.

Similarly, the shielding member 05 is hollow inside, and a slot 07 for fixing the pump head is provided on the outer side of the top of the shielding member 05. The shielding member 05 is sleeved on the outer part of the first opening 03, and the shielding member 05 and the housing 01 form a sleeving portion 06 at the overlapping sleeving part. A first space 08 is reserved inside the shielding member 05 above the housing 01.

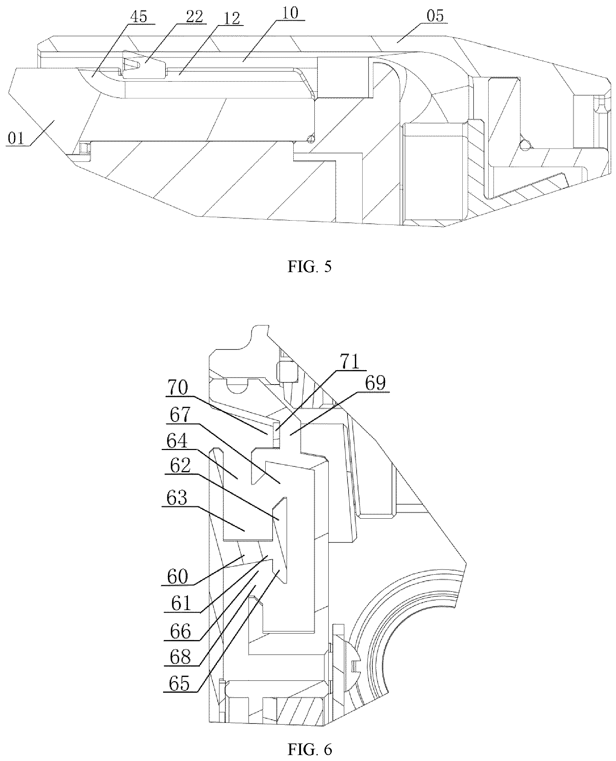

With reference to FIG. 4 and FIG. 5, a first gap 10 is provided between the housing 01 and the shielding member 05 at the sleeving portion 06, and the first space 08 is in communication with the first gap 10.

In the embodiments of the present application, the first gap is provided between the housing and the shielding member, which allows communication between the inside and the outside, enabling the exchange of gas between the inside and outside. From a cross-sectional perspective, the first gap forms an annular channel for internal and external gas exchange, increasing the contact area for gas heat exchange.

The lower part of the rotating member 09 is installed in the first cavity 02 and is surrounded by the sleeving portion 06, and the upper part of the rotating member 09 is accommodated in the first space 08. The rotating member 09 is used to disturb the airflow in the first space 08 by rotation.

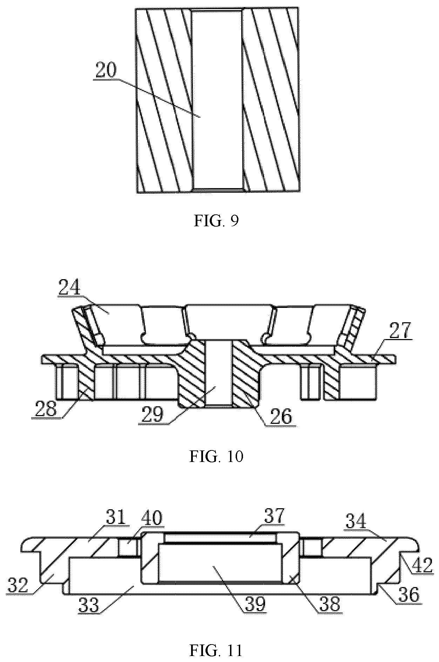

With reference to FIG. 3, FIG. 4, FIG. 8, and FIG. 9, the rotating member 09 includes: a stator 13, a rotor 14, a motor shaft 15, and a rotating platform 16.

Further, the stator 13 is installed in a mounting slot 17 within the first cavity 02, and the stator 13 is provided with a metal winding. A side of the stator 13 is in contact with the inner surface of the first cavity 02. The rotor 14 is rotatably installed in a second cavity 18 formed as a hollow inside the stator 13, and a third gap 19 is reserved between the rotor 14 and the inner wall of the second cavity 18.

In the embodiments of the present application, an elastic member 22 is provided between the stator 13 and the mounting slot 17. The elastic member 22 is compressed to produce deformation, which helps eliminate assembly tolerances and optimize coaxiality.

As described above, the stator 13 and the rotor 14 are installed at the position of the sleeving portion 06, separating the first cavity 02 and the first space 08. Since a third gap 19 is reserved between the rotor 14 and the inner wall of the second cavity 18, the first cavity 02 and the first space 08 are in communication. The airflow entering from the first cavity 02 can flow into the first space 08 through the third gap 19.

The motor shaft 15 is inserted into a third cavity 20 formed as a hollow inside the rotor 14. Bearings 21 are sleeved on both ends of the motor shaft 15 extending out from the third cavity 20. The rotating platform 16 is installed at the top end of the motor shaft 15 and is located within the first space 08 below the slot 07. The rotor 14 can rotate within the second cavity 18, and the rotation of the rotor 14 can drive the rotating platform 16 to rotate together.

In the embodiments of the present application, the stator and rotor are the main heat-generating sources of the rotating member. The first gap is provided on the outer side of the rotor and stator. The first gap not only serves as a passage allowing airflow to flow through, but also plays a role in heat dissipation on the outer side of the main heat-generating sources, thereby improving heat dissipation efficiency.

Further, with reference to FIG. 3, FIG. 4, and FIG. 10, the rotating platform 16 includes: an adapting portion 24, a blade assembly 25, a first receiving portion 26, and a first extending portion 27.

The adapting portion 24 is located on a side of the rotating platform 16 near the slot 07. At least one magnetic member (not shown in the figures) is provided inside the adapting portion 24. The adapting portion 24 is magnetically connected to the pump head by the magnetic member, so that the rotation of the rotating platform 16 drives the pump head mounted on the slot 07 to rotate together.

The blade assembly 25 is located on a side of the rotating platform 16 away from the slot 07, and the blade assembly 25 has a plurality of blades 28.

In the embodiments of the present application, by adding blades 28, greater airflow can be generated during rotation. Moreover, the faster the rotation, the more heat is generated by the stator 13, and the faster the airflow, the quicker the heat is dissipated, thereby achieving temperature balance or enabling the overall temperature to vary little within a specified power range. The number of blades 28 is greater than or equal to 11 and less than or equal to 15. The number of blades 28 is preferably 11.

Further, in the embodiments of the present application, the blades 28 are arc-shaped, and the arc orientation of the blades 28 is consistent with the rotation direction of the rotating platform 16. The blade design with this arc shape and arc orientation can maximize the air output.

The first receiving portion 26 is located in the middle of the rotating platform 16 and has a first through hole 29 for receiving the top end of the motor shaft 15.

The first extending portion 27 is formed by extending outward from the junction of the adapting portion 24 and the blade assembly 25, and a fourth gap 30 is provided between the outer end of the first extending portion 27 and the inner wall of the shielding member 05.

Gas sequentially enters from the second opening 04 into the first cavity 02 and the first space 08, and is then discharged through the first gap 10.

The basic modes of heat transfer include thermal conduction, thermal convection, and thermal radiation. Among them, thermal conduction refers to the process in which heat is transferred from the higher-temperature part of an object to the adjacent lower-temperature part, or from a high-temperature object to a low-temperature object in contact with it, under conditions involving material transfer. Thermal convection refers to the heat exchange caused by the relative motion of different parts of a fluid at different temperatures. Thermal radiation refers to the phenomenon in which an object radiates energy due to its own temperature.

In the field of medical devices, heat conduction is commonly used for heat dissipation. By filling a thermally conductive medium between the heat-generating components and the housing, the heat from the heat-generating components is transferred to the housing. The air in direct contact with the housing can carry away some of the heat from the housing. However, this heat dissipation method relies solely on the thermally conductive medium, and due to the limited thermal conductivity of the medium, it can only transfer part of the heat from the heat-generating components to the housing, resulting in insufficient heat transfer. Furthermore, as the air in contact with the housing flows slowly, its heat dissipation efficiency is low. This heat dissipation method, which relies only on the thermally conductive medium, typically improves heat dissipation efficiency by increasing the application area of the thermally conductive medium, such as by adding fins, grooves, etc., to the heat-generating components.

As described above, in the embodiments of the present application, the main method of accelerating the air circulation within the device cavity is through thermal convection, which more efficiently transfers the heat away.

Moreover, in existing ECMO devices, the motor is generally protected by a housing. In the embodiments of the present application, the motor housing is removed, and the stator is directly in contact with the inner wall of the housing, so that the largest heat source first transfers heat to the pump driving housing through thermal conduction, while some of the heat is carried away by thermal convection. The airflow from convection then passes over the largest heat source at the pump driving housing, carrying away more heat.

Further, a plurality of recessed channels 11 are provided on the outer side of the sleeving portion 06 of the housing 01, and the shielding member 05 is sleeved on the recessed channels 11. A second gap 12 is formed between the bottom of each recessed channel 11 and the shielding member 05. The first space 08 is in communication with the second gap 12.

The plurality of recessed channels 11 covers the area where the stator 13 contacts the housing 01. The stator 13 is the largest heat source, and it is in contact with the inner wall of the housing 01. The corresponding position where the stator 13 and the housing 01 are in contact (i.e., the sleeving portion 06) is where the heat transfer is most efficient. By providing the plurality of recessed channels 11, the surface area in this region is expanded, which further aids in heat dissipation. The airflow disturbed by the rotating member is directed through the sleeving portion and then flows into the recessed channels, passing through the second gap and then the first gap before being discharged. This combination design accelerates heat dissipation in this region, making the heat dissipation more efficient.

The plurality of recessed channels 11 extend along the area where the stator 13 is in contact with the inner surface of the first cavity 02, and they are arranged to encircle the first cavity 02. The recessed channels 11 cover the area where the stator 13 contacts the housing 01. The surrounding arrangement results in varying depths of the recessed channels 11, which helps maximize the increase in heat dissipation surface area. Each recessed channel 11 is in communication with the first gap 10 and the first space 08. In this way, gas sequentially enters the first cavity 02 and the first space 08 from the second opening 04 and is then discharged through the first gap 10 and the second gap 12.

With reference to FIG. 2 and FIG. 4, a motor blocking member 23 is provided at an end where the stator 13 and rotor 14 away from the mounting slot 17. The motor blocking member 23 covers the first opening 03 and is used to restrict the stator 13 within the first cavity 02.

Specifically, with reference to FIG. 2, FIG. 4, and FIG. 11, the motor blocking member 23 includes: a first body 31, a docking portion 32, a second receiving portion 33, and a second extending portion 34.

The first body 31 is located below the rotating platform 16 and a fifth gap 35 is formed between it and the bottom ends of the blades 28.

The docking portion 32 is located at an edge of the first body 31 and extends towards the housing 01. At the location where it interfaces with the outer end of the first opening 03, a first step 36 is provided.

The second receiving portion 33 is located at the center of the first body 31 and has a second through hole 37 that receives the first receiving portion 26. Surrounding the second through hole 37, a protruding portion 38 extends towards the first cavity 02, forming a second space 39 to accommodate the bearing 21. A plurality of through slots 40 can be provided on the outer edge of the protruding portion 38, with no blade 28 obstructing above each through slot 40.

The second extending portion 34 extends outward from an edge of the first body 31 at the junction with the docking portion 32. A sixth gap 41 is formed between the outer end of the second extending portion 34 and the inner wall of the shielding member 05. The docking portion 32 and the second extending portion 34 form a second step 42 and a first annular groove 43.

As described above, in the embodiments of the present application, the rotating platform 16 is located above the motor blocking member 23, and there is a fifth gap 35 between the motor blocking member 23 and the blade 28. The motor blocking member 23 has a plurality of through slots 40 around the protruding portion 38, with no blade 28 obstructing above each through slot 40. This design allows heat that rises from the bottom to be directly carried away by the rotation of the blades 28, making the treatment of hot air more effective.

In addition, the first annular groove 43 is an annular drainage channel formed within the first space 08. Liquid flows into it from one side through the first gap 10 and the second gap 12, then falls into the first annular groove 43, and is then discharged out through the other side through the second gap 12.

Further, on the basis that the second gap 12 is present, a sealing member 44 is provided between the housing 01 and the shielding member 05, and the sealing member 44 is used to block the first gap 10. In this way, the airflow entering the first cavity 02 and the first space 08 can be discharged through the second gap 12.

In the embodiments of the present application, the sealing member 44 is provided to block the first gap 10, with the purpose of directing the flow of liquid toward a designated direction with the sealing member 44. More specifically, it ensures that the inflowing liquid can only enter from the bottom of the recessed channels, preventing it from entering the pump driving device interior by crossing the first step from the upper part of the recessed channel.

Each recessed channel 11 has a bottom facing the second opening 04 and is provided with an arc-shaped transition section 45 on the outer side of the housing 01. The sealing member 44 is disposed between the arc-shaped transition section 45 and the first opening 03, which makes the inflow or outflow of liquid more convenient and provides a certain drainage effect.

The height of the first gap 10 is less than the first distance between the bottom of the sealing member 44 and the bottom of the recessed channel 11, and the first distance is less than the height of the second extending portion 34. In this way, it prevents excessive incoming liquid from overflowing due to insufficient drainage or forming a vortex and overflowing.

The second distance between the bottom of each recessed channel 11 and the central axis of the first space 08 is greater than or equal to the third distance between the bottom of the first annular groove 43 and the central axis of the first space 08, thereby preventing water accumulation in the channel.

Compared with the existing technology, the pump driving device provided in the embodiment of the present application includes a shielding member 05 sleeved on the outer side portion of the first opening 03 of the housing 01 to form a sleeving portion 06. A first space 08 is reserved inside the shielding member 05 above the housing 01. A first gap 10 and a second gap 12 are provided in the sleeving portion 06 between the housing 01 and the shielding member 05, so that the first space 08 is in communication with the first gap 10 and the second gap 12. A rotating member 09 rotates within the first space 08 to disturb the airflow and accelerate the internal gas flow, and the first cavity 02 is in communication with the first space 08. In this way, gas sequentially enters the first cavity 02 and the first space 08 from the second opening 04, and is discharged through the first gap 10 and the second gap 12, forming an effective airflow heat dissipation channel. Moreover, the positions of the first gap 10 and the second gap 12 are located on the outer side of the main heat source, i.e., the rotating member 09, thereby improving heat dissipation efficiency. In addition, the first gap 10, the second gap 12, and the first annular groove 43 also form a water drainage channel, simultaneously meeting the requirements for heat dissipation and waterproofing.

In the patent application with publication number CN116231963 A, a circular groove is formed by a downward recess at only one end for gas exchange with the outside, and the structure and position of the airflow exchange outlet result in limited gas exchange efficiency. Moreover, the arrangement and combination of internal heat-generating components, the stator, and the housing with the heat dissipation channel also lead to a large amount of heat energy not being effectively transferred out. A thermal equilibrium state refers to a state in a closed system where all parts reach the same temperature, and there is no net heat flow within the system. In the thermal equilibrium state, the temperature distribution in the system no longer changes with time, meaning that the heat input and output have reached balance. Under the same conditions, in comparative tests conducted in the thermal equilibrium state, compared with the design in the embodiment of the patent application with publication number CN116231963 A, the embodiment of the present application achieves a temperature reduction of 4°C in both the bearing temperature and the winding temperature at the stator.

In combination with Figures 2, 4, and 6, the pump driving device disclosed in the embodiment of the present application further includes: a closing member 46 and a cover plate 47. The closing member 46 is covered on the second opening 04 at the bottom end of the pump driving device, and the cover plate 47 is mounted outside the closing member 46. A third space 48 with an external opening is formed between the closing member 46 and the cover plate 47.

Furthermore, the closing member 46 includes: a bottom panel 49 and a side panel 50. The bottom panel 49 is used to cover the second opening 04, and a vent hole 51 is provided on the bottom panel 49. The side panel 50 is formed by outwardly folding from the edge of the bottom panel 49, and the outer edge of the side panel 50 is connected to the outer end of the second opening 04.

Further, the vent hole 51 may be circular or square, as shown in FIG. 4. If a speaker is mounted at the center of the bottom panel 49, the vent hole 51 may also be annular, in which case the vent hole 51 is arranged around the center of the bottom panel 49.

At least one protrusion provided on the inner surfaces of the closing member 46 and the cover plate 47 facing the third space 48 forms an annular flow guide channel, and the sum of the height of the protrusion on one side and the height of the protrusion on the other side is greater than the fourth distance between the closing member 46 and the cover plate 47.

Each protrusion is arranged in an annular shape. The pump driving device in the embodiments of the present application can be used in an inclined placement based on the actual application scenario, with the inclined placement angle being greater than or equal to 0° and less than or equal to 180°, and the most common placement direction being an inclined placement angle equal to 90°. In the case where a protrusion is present only on the second opening side, liquid splashed into the third space will drip onto the protrusion. Since the protrusion is annular, the liquid flows downward along the annular protrusion under the action of gravity, and is subsequently discharged along the third space. However, in this case, if the protrusion height is insufficient and there remains a relatively large gap, the liquid may still enter the second opening at a certain angle; if the height is too great, the opening becomes smaller, which is unfavorable for gas exchange inside the pump driving device. When both sides are provided with protrusions and the sum of their heights is greater than the fourth distance, it is possible to prevent liquid from entering the second opening at any angle, while still allowing a relatively large opening for gas exchange between the inside and outside of the pump driving device, thereby achieving both good waterproof performance and heat dissipation effect.

Preferably, in the embodiments of the present application, an outward protruding portion is provided on the side surface of the protrusion. On the one hand, the outward protruding portion provided can prevent the liquid flowing on the surface of the protrusion from dripping into the third space before being discharged in time, thereby enhancing the water drainage capability. On the other hand, the outward protruding portion can prevent the liquid that drips onto the protrusion from splashing into the third space.

In the embodiments of the present application, the protrusion includes: a first annular protrusion 52, and the first annular protrusion 52 surrounds the outer edge of the vent hole 51. The first annular protrusion 52 serves as the protrusion, and the outward protruding portion is provided on the side surface of this protrusion. The outer end of the first annular protrusion 52 extends and folds toward the side panel 50 to form a second annular protrusion 53. The second annular protrusion 53 is the outward protruding portion of the first annular protrusion 52. The first annular protrusion 52 having the second annular protrusion 53 forms a second annular groove 54 with the bottom panel 49.

The side panel 50 includes an inner side plate 55 and an outer side plate 56. The protrusion also includes: a third annular protrusion 57 formed by folding the outer end of the inner side plate 55 inward, with the third annular protrusion 57 being arranged opposite to the second annular protrusion 53. The inner side plate 55 with the third annular protrusion 57 forms a third annular groove 58 with the bottom panel 49; the second annular groove 54 and the third annular groove 58 are connected by the bottom panel 49 to form a first annular recess 59.

The protrusion also includes: a fourth annular protrusion 60 formed by extending the inner side surface of the cover plate 47 inward, with the fourth annular protrusion 60 surrounding the first annular protrusion 52. The fourth annular protrusion 60 extends into the first annular recess 59 and forms an in-recess extending portion 61. The fourth annular protrusion 60 serves as the protrusion, and the outward protruding portion is provided on the side surface of this protrusion. The in-recess extending portion 61 extends and flips toward the third annular groove 58 to form a fifth annular protrusion 62, with the fifth annular protrusion 62 being the outward protruding portion of the fourth annular protrusion 60. The fourth annular protrusion 60 with the fifth annular protrusion 62 forms a fourth annular groove 63 with the cover plate 47. The fourth annular groove 63 is arranged opposite to the outer side plate 56 with the third annular protrusion 57, forming a second annular recess 64.

The outer end of the fifth annular protrusion 62 and the inner side plate 55 have a seventh gap 67 between them, and the outer end of the second annular protrusion 53 and the fourth annular protrusion 60 have an eighth gap 68 between them.

The width of the fourth annular groove 63 is greater than the height of the fifth annular protrusion 62, and the height of the fifth annular protrusion 62 is greater than the fifth distance between the outer end of the fifth annular protrusion 62 and the outer end of the third annular protrusion 57. The fifth distance is greater than the height of the third annular protrusion 57.

In the embodiments of the present application, the accumulation of water to a certain height is allowed. This proportional arrangement is more favorable for drainage, allowing a large amount of water to enter from the inlet, while the structure can still discharge it smoothly.

The in-recess extending portion 61 extends and flips toward the second annular groove 54 to form a sixth annular protrusion 65, with the sixth annular protrusion 65 also being the outward protruding portion of the fourth annular protrusion 60. The fourth annular protrusion 60 with the sixth annular protrusion 65 forms a third annular recess 66 with the cover plate 47.

The inner side plate 55, outer side plate 56, and fourth annular protrusion 60 are all inclined toward the side near the first annular protrusion 52, facilitating the drainage of liquid. The liquid can flow along the inclined surfaces, preventing it from dripping into the device when it reaches the center.

A transition plate 69 is provided between the inner side plate 55 and the outer side plate 56. One end of the transition plate 69 is connected to the inner end of the outer side plate 56, and the other end of the transition plate 69 is connected to the outer side of the inner side plate 55. The outer side plate 56, transition plate 69, and inner side plate 55 form a fifth annular groove 70, within which a splash pad 71 is provided.

In the embodiments of the present application, a splash pad is provided to allow the liquid that is sprayed in to undergo diffuse reflection, thereby reducing the water flow rate and decreasing the subsequent water drainage pressure.

Waterproofing methods are generally divided into three types: sealed waterproofing, shielding, and drainage. Fully sealed waterproofing provides good waterproofing effects, but the internal heat can only be dissipated through the housing, which results in poor heat dissipation efficiency. Shielding waterproofing has poor waterproofing effects and is prone to infiltration of liquid. A single drainage-type waterproofing method may not be able to expel the liquid in time.

As described above, in the embodiments of the present application, an annular flow guide channel is formed at the second opening by providing at least one protrusion on the inner surfaces of the closing member and cover plate facing inward. When the second opening is rained during severe weather, liquid flows into the annular flow guide channel from one side and can be guided to flow out from the other side. The embodiments of the present application adopt a combination of shielding and drainage for waterproofing. The key feature of this arrangement is the separation of water and vapor. Considering outdoor rainy conditions and the application scenario of the pump driving device, whether placed horizontally, vertically, or inclined between the two, it can prevent liquid from entering to a certain extent. If liquid does enter, it can be expelled through drainage. The device can meet the IPX4 waterproof requirement. It does not affect the internal heat dissipation while ensuring waterproofing.

Although the present invention has been described in detail with general descriptions and specific embodiments, modifications or improvements can be made based on the invention, which would be apparent to a person skilled in the art. Therefore, any modifications or improvements made without departing from the principles of the invention are within the scope of protection of the present invention.

## Claims

1. A pump driving device, comprising:
a housing, which is hollow inside to form a first cavity, and has a first opening and a second opening respectively formed at two ends thereof;
a shielding member, which is sleeved on an outer side of the first opening and forms a sleeving portion with the housing, with a slot provided at an outer side of a top of the shielding member for fixing a pump head, and a first space is reserved inside the shielding member on top of the housing; and
a rotating member, configured to disturb an airflow by rotation within the first space, with the first cavity and the first space being in communication, wherein
a first gap is formed between the housing and the shielding member at the sleeving portion, and a plurality of recessed channels are provided on an outer side of the sleeving portion of the housing;
gas enters the first cavity and the first space sequentially from the second opening, and is then discharged through the first gap and the plurality of recessed channels.

2. The pump driving device according to claim 1, wherein a second gap is formed between bottoms of the plurality of recessed channels and the shielding member; and the first space is in communication with the first gap and the second gap.

3. The pump driving device according to claim 1 or 2, wherein the rotating member comprises:
a stator, mounted in a mounting slot within the first cavity, with a side of the stator in contact with an inner surface of the first cavity;
a rotor, rotatably mounted within a second cavity formed by the stator being hollow, with a third gap formed between the rotor and an inner wall of the second cavity;
a motor shaft, inserted into a third cavity formed by the rotor being hollow, with both ends of the motor shaft extending from the third cavity sleeved with bearings; and
a rotating platform, mounted at a top end of the motor shaft and located within the first space below the slot.

4. The pump driving device according to any of claims 1 to 3, wherein the plurality of recessed channels extend along a junction between the stator and an inner surface of the first cavity and are arranged around the first cavity, with each recessed channel being in communication with the first gap and the first space.

5. The pump driving device according to any of claims 1 to 4, wherein a motor blocking member is provided at an end where the stator and the rotor are away from the mounting slot, the motor blocking member covers the first opening, and the motor blocking member is configured to confine the stator within the first cavity.

6. The pump driving device according to any of claims 3 to 5, wherein the rotating platform comprises:
an adapting portion, located on a side of the rotating platform near the slot, the adapting portion being internally provided with at least one magnetic member;
a blade assembly, located on a side of the rotating platform away from the slot, having a plurality of arc-shaped blades;
a first receiving portion, located in a middle portion of the rotating platform, and having a first through hole for receiving the top end of the motor shaft; and
a first extending portion, formed by extending outward from a junction between the adapting portion and the blade assembly, a fourth gap being formed between an outer end of the first extending portion and an inner wall of the shielding member.

7. The pump driving device according to claim 5 or 6, wherein the motor blocking member comprises:
a first body, located below the rotating platform and having a fifth gap between the first body and bottom ends of the blades;
a docking portion, provided at an edge of the first body and extending toward the housing, with a first step being formed at a docking position with an outer end of the first opening;
a second receiving portion, located in a middle portion of the first body, having a second through hole for receiving the first receiving portion, and a protruding portion formed extending toward the first cavity around a periphery of the second through hole, wherein the protruding portion encloses a second space for accommodating the bearings, and a plurality of through slots are provided on an outer periphery of the protruding portion; and
a second extending portion, formed by extending outward from an edge of the first body at a junction with the docking portion, wherein a sixth gap is formed between an outer end of the second extending portion and the inner wall of the shielding member, and the docking portion and the second extending portion form a second step and a first annular groove.

8. The pump driving device according to any of claims 1 to 7, wherein an arc-shaped transition section is provided on an outer side of the housing at an end of a bottom of each recessed channel facing the second opening, and a sealing member is provided between the arc-shaped transition section and the first opening.

9. The pump driving device according to any one of claims 1 to 8, wherein the pump driving device further comprises: a closing member and a cover plate, the closing member is mounted to cover the second opening at a bottom of the pump driving device, the cover plate is arranged outside the closing member, a third space with an outward opening is formed between the closing member and the cover plate, at least one protrusion facing the third space is arranged on the closing member and an inner surface of the cover plate to form an annular flow guide channel, and a sum of a height of one protrusion on one side and a height of another protrusion on another side is greater than a fourth distance between the closing member and the cover plate.

10. The pump driving device according to claim 9, wherein the closing member comprises:
a bottom panel, configured to cover the second opening, with a vent hole provided on the bottom panel; and
a side panel, formed by folding an edge of the bottom panel outward, with an outer edge of the side panel connected to an outer end of the second opening.

11. The pump driving device according to claim 10, wherein the protrusion comprises: a first annular protrusion, the first annular protrusion surrounds an outer edge of the vent hole, an outer end of the first annular protrusion is folded and extends toward the side panel to form a second annular protrusion, and the first annular protrusion having the second annular protrusion and the bottom panel form a second annular groove.

12. The pump driving device according to either of claims 10 and 11, wherein the side panel comprises an inner side plate and an outer side plate, and the protrusion further comprises a third annular protrusion formed by an outer end of the inner side plate being folded inward and extending, the third annular protrusion is disposed opposite to the second annular protrusion; the inner side plate having the third annular protrusion and the bottom panel form a third annular groove; and the second annular groove and the third annular groove are connected via the bottom panel to form a first annular recess.

13. The pump driving device according to any of claims 9 to 12, wherein the protrusion further comprises a fourth annular protrusion formed by an inner side surface of the cover plate extending inward, the fourth annular protrusion surrounds an outer side of the first annular protrusion, the fourth annular protrusion extends into the first annular recess to form an in-recess extending portion, the in-recess extending portion is folded and extends toward the third annular groove to form a fifth annular protrusion, the fourth annular protrusion having the fifth annular protrusion and the cover plate form a fourth annular groove, and the fourth annular groove and the outer side plate having the third annular protrusion are disposed oppositely to form a second annular recess.

14. The pump driving device according to claim 13, wherein the inner side plate, the outer side plate, and the fourth annular protrusion are all obliquely arranged on a side close to the first annular protrusion.

15. The pump driving device according to any of claims 9 to 14, wherein a transition plate is provided between the inner side plate and the outer side plate, one end of the transition plate is butted with an inner end of the outer side plate, and another end of the transition plate is connected to an outer side of the inner side plate, and the outer side plate, the transition plate, and the inner side plate together enclose a fifth annular groove, and a splash pad is provided in the fifth annular groove.
